# EUROPÄISCHE PATENTSCHRIFT

(11) **EP 2 922 467 B1**
(45) Veröffentlichungstag und Bekanntmachung des Hinweises auf die Patenterteilung: **26.10.2022**
(21) Anmeldenummer: 13805746.8
(22) Anmeldetag: 15.11.2013
(51) Int. Cl.: A61B 5/055, A61B 5/00, G01R 33/34, G01R 33/3415

(54) **SPULENANORDNUNG FÜR EIN MAGNETRESONANZTOMOGRAPHISCHES GERÄT**
COIL ARRANGEMENT FOR A MAGNETIC RESONANCE IMAGING DEVICE
ENSEMBLE DE BOBINES POUR UN APPAREIL TOMOGRAPHIQUE À RÉSONANCE MAGNÉTIQUE

(30) Priorität: 22.11.2012 DE 102012022779
(43) Veröffentlichungstag der Anmeldung: 30.09.2015
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2013/100386
(87) Internationale Veröffentlichungsnummer: WO 2014/079416

(56) Entgegenhaltungen:
- DE-A1- 10 221 644
- DE-A1- 10 317 629
- DE-A1-102011 075 440
- US-A1- 2010 253 351

## Beschreibung

Die Erfindung bezieht sich auf ein magnetisches Resonanz-tomographisches Gerät für Prostatauntersuchungen an einem darin befindlichen Patienten
gemäß des Oberbegriffs des Anspruchs 1.

Frühzeitige Erkennung von insbesondere Prostatakrebs ist wichtig für eine erfolgreiche Behandlung. Die normalen Methoden zur Suche nach Prostatakrebs wie manuelle Untersuchungen und Bluttests versagen bei der Auffindung von manchen bösartigen Tumoren oder geben manchmal fälschlicherweise ein positives Testergebnis. Biopsie ist der Weg zur Auffindung eines Tumors und zur Bewertung von dessen Gefährlichkeit. Leider verfehlt diese Biopsie oft den Tumor.

Die magnetische Resonanz-Tomographie ist ein bildgebendes Verfahren mit exzellentem Weichteilkontrast, Knochen hingegen werden nicht scharf abgebildet. Deshalb wird es für die Untersuchung der Prostata eingesetzt, wobei eine möglichst gute Auflösung erzielt werden soll.

Die magnetische Resonanz-Tomographie basiert auf den Prinzipien der Kernspinresonanz (NMR), insbesondere der Feldgradienten-NMR, und wird daher auch als Kernspintomographie bezeichnet. Mit Hilfe der magnetischen Resonanz-Tomographie kann man Schnittbilder des menschlichen (oder tierischen) Körpers anfertigen, die eine Beurteilung der Organe und vieler krankhafter Organveränderungen möglich machen. Die magnetische Resonanz-Tomographie erfordert ein sehr starkes statisches Magnetfeld sowie elektromagnetische Wechselfeldern im Radiofrequenzbereich, mit denen bestimmte Atomkerne (eigentlich immer die Wasserstoffkerne) im Körper resonant angeregt werden, welche dann emittiert und im Empfängerstromkreis elektrische Signale induzieren. Zur Erreichung der Ortsauflösung werden Gradientenspulen verwandt, die zur Erzeugung der magnetischen Gradientenfelder dienen. Die Gradientenspulen werden paarweise mit gleicher Stromstärke aber gegensinniger Polung benutzt, sodass die eine Spule das statische magnetische Feld verringert, die gegenüberliegende Spule hingegen, es um den gleichen Betrag erhöht. Dadurch wird im Ergebnis das magnetische Feld mit einem linearen Gradienten versehen. Für alle drei Raumrichtungen gibt es so eine Vorrichtung. Hintergrund ist, dass das lokale magnetische Feld die Resonanzfrequenz bestimmt und dadurch eine räumliche Lokalisierung möglich macht. Nach dem Stand der Technik werden normalerweise flächige Empfangsspulen verwendet, d.h. mindestens ein flächiges Spulenelement liegt unter dem Rumpf des Patienten und ein flächiges Spulenelement liegt auf dem Patienten. Typischerweise bestehen diese Spulenelemente jeweils aus sechs Spulen, wobei meist zu jeder Spule ein Vorverstärker vorhanden ist. Diese sogenannten Phased-Array Spulen sind eine Kombination von mehreren Oberflächenspulen zu einem Array. Die Idee ist, mit relativ kleinen Spulen, welche ein gutes Signal- zu Rausch-Verhältnis aufweisen, trotzdem ein großes Gebiet abdecken zu können. Das Rauschen setzt sich aus dem thermischen Rauschen des Messobjektes und dem thermischen Rauschen der Hochfrequenzspule zusammen.

Jedoch sind diese Empfangsspulen baulich bedingt relativ weit von der Prostata entfernt. Dadurch ist nur eine unzulängliche Auflösung der Prostata gewährleistet. Des Weiteren wird zur Verbesserung der räumlichen Auflösung bereits heute eine sog. Endorektalspule genutzt, die eine bessere räumliche Auflösung der Prostata erlaubt, da hier die Spule in unmittelbarer Nähe zur Prostata positionierbar ist.

Die Offenlegungsschrift DE 103 17 629 A1 offenbart ein magnetresonanz-tomographisches Gerät für Prostatauntersuchungen mit einem darin befindlichen Patienten, welches auf einer Spule zur Erzeugung eines starken homogenen magnetischen Feldes in Richtung der Längsachse des Patienten, mindestens einer Sendespule zur Erzeugung eines elektromagnetischen Wechselfeldes, drei Gradientenspulen, aus einer Datenverarbeitung zur Bildgewinnung aus den Signalen der Sende- und Empfangsspulen, sowie geeigneten Empfangsspulen besteht, von denen einzelne unterhalb in der unteren Rückenregion und/oder am Hinterteil und wenigstens eine oberhalb des Patienten angeordnet sind.

Die Offenlegungsschrift DE 102 21 644 A1 offenbart ebenfalls eine Lokalspulenanordnung für eine Magnetresonanzanlage, welche Empfangsspulen umfasst, von denen einzelne unterhalb in der unteren Rückenregion und/oder am Hinterteil und wenigstens eine oberhalb des Patienten angeordnet sind.

Aufgabe der Erfindung ist es, ein Gerät zu schaffen, bei dem ohne eine Endorektalspule einsetzen zu müssen, dennoch die räumliche Auflösung der Prostata entscheidend erhöht wird.

Die Lösung dieser Aufgabe besteht darin, dass ein Spulenelement vorgesehen ist, das in drei dreiecksförmig angeordnete Teilspulen unterteilt ist und so eingerichtet ist, dass, wenn sich zwei der Teilspulen parallel nebeneinander auf der unteren Bauchregion des Patienten befinden, eine dritte, geschlossene und mittig angefügte V-förmige Empfangsspule den Hodensack und den Penis umschließt und an den Leisten des Patienten anliegt, wobei Hodensack und Penis eine Öffnung der Empfangsspule durchgreifen.

Dadurch wird die Spule in unmittelbarer Nähe der Prostata positioniert und erlaubt somit eine bessere räumliche Auflösung, da sie mehr Signal vom Volumenelement Prostata auffangen kann. Diese neuartige Spule wird in Kombination mit einem herkömmlichen flächigen Spulenelement am Rücken des Patienten eingesetzt. Eigentlich müsste man den Patienten von allen Seiten mit Empfangsspulen umgeben, um möglichst viel Signal aufzufangen, d.h. auch seitlich wären Empfangsspulen sinnvoll. Dies ist jedoch aufgrund der unterschiedlichen Anatomie der Körper der Patienten praktisch schlecht umsetzbar. Beim schlanken Patienten wird ein größerer Umfangswinkel vom Standardspulenelement abgedeckt, d.h. die Signalmenge, die verloren geht, ist kleiner, da auch der Abstand zwischen Ober- und Unterseite des Patienten kleiner ist; so wird hier eine bessere Auflösung erreicht. Beim dickeren Patienten ist der vom Standardspulenelement abdeckte Umfangswinkel kleiner, d. h. hier geht mehr Signal seitlich verloren. Folglich ist die Auflösung schlechter als beim schlanken Patienten. Die naheliegende Lösung wäre für unterschiedliche Patienten unterschiedliche Standardspulenelemente vorzusehen, was jedoch nicht praktikabel ist.

Eine optimale Bildqualität lässt sich dann erreichen, wenn die die Hoden umgebende Empfangsspule mit ihrer Flächennormalen in Richtung der Prostata ausgerichtet ist. Der Begriff "Flächennormale" ist bereits bei ebenen Spulen nicht eindeutig, da eine Vielzahl von parallel zueinander ausgerichteten Flächennormalen existiert. Bei gekrümmten Spulen gilt weiterhin, dass die in den einzelnen Punkten der Fläche senkrecht, also senkrecht zu der dort verlaufenden Tangentialebene definierten Flächennormalen, unterschiedliche Orientierungen aufweisen, soll zur eindeutigen Bestimmung des Verlaufs der Flächennormalen jene ausgewählt werden, die durch den Flächenschwerpunkt der Empfangsspule verläuft, sodass im Ergebnis eine eindeutige Anweisung zum Handeln vorliegt.

Erfindungsgemäß werden oberhalb des Patienten drei Spulen dreiecksförmig angeordnet, d.h. dass zwei Spulen parallel nebeneinander auf dem Bauch des Patienten anliegen und die dritte Spule den Hodensack und den Penis umschließt. Die dritte Spule liegt mit den unteren Längsseiten V-förmig an der rechten und linken Leiste an, sodass der Hodensack und der Penis die Öffnung der Spule durchgreifen. Durch das Spreizen der Oberschenkel und mit leichtem Andruck der Spule bekommt man eine optimale Ausrichtung des Spulenelementes. Die beiden oberen Spulenelemente befinden sich zwischen der Leiste und der unteren Bauchdecke und können mit ihrer Flächennormale in Richtung Prostata ausgerichtet sein.

Gem. einer Alternativen kann die magnetische Resonanz-Tomographie-Spule auch aus mehr als drei Spulen an der Oberseite des Patienten aufgebaut sein. Dadurch kann das Auflösungsvermögen erhöht werden, da das Signal- zu Rausch-Verhältnis bei kleineren Spulen günstiger wird. Die Anzahl der Spulen definiert in ihrer Gesamtheit jene Fläche, in der Signale erfasst und damit auch ausgewertet werden können. Dabei bestimmt die äußere Umrandung dieser Fläche die sich als Summe der Flächen der einzelnen Spulen addiert, die Eindringtiefe der gesamten Anordnung, die aus allen Spulen gebildet wird. Werden mehrere Spulen zusammen und gleichzeitig eingesetzt, erhält man ein Messergebnis, bei dem die hohe Empfindlichkeit der einzelnen Spule zum einen und die hohe Eindringtiefe der gesamten Anordnung zum anderen kombiniert werden.

Die relative Zuordnung der Spulen ist im Rahmen der Erfindung grundsätzlich beliebig. So können die Spulen zueinander beabstandet sein, was durch bauliche Zwänge geboten sein kann. Als nachteilig ist anzusehen, dass die in Zwischenräume der Spule emittierten Signale nicht genutzt werden können.

In einem bevorzugten Fall schließen die Spulen direkt aneinander an, was den Vorteil hat, dass möglichst wenig emittierte Signale verloren gehen. Je höher die empfangene Intensität der Signale, desto besser wird die Bildqualität.

Das Auflegen und Entfernen wird stark vereinfacht, wenn die Spulen in einer flexibeln Matte untergebracht sind. Dies hat den Vorteil, dass sich diese Matte weitgehend der individuellen Körperform des Patienten anpasst. Damit liegen die Empfangsspulen möglichst dicht am Patienten an und erlauben somit eine bessere Bildqualität. Ein Durchgriff für Penis und Hodensack muss in der Matte vorhanden sein.

Ziel ist es, das die Prostata umgebende und darstellende Volumenelement scharf abzubilden. Zur Optimierung ist der Radius der Empfangsspule so gewählt, dass er größer oder gleich dem durchschnittlichen Abstand der Spulenebene zum zu untersuchenden Organ, hier der Prostata, ist.

Der Abstand variiert von Patient zu Patient, deshalb wird hier der Begriff "durchschnittlicher Abstand" als Mittelwert der anatomischen Gegebenheiten beschrieben. Die Eindringtiefe hängt direkt vom Spulenradius ab. Die Auflösung ist optimal, wenn die Entfernung des zu untersuchenden Organs von der Spulenebene maximal dem Radius der Spule entspricht. Grundsätzlich gilt, dass die Bildqualität umso besser wird, je geringer der Abstand Spule-Prostata ist.

Es wurde als empfehlenswert erkannt, während der Aufnahmephase den Patienten im Bereich des Bauches oder Rumpfes zumindest mit einer diese teilweise umschließende Fixiereinrichtung räumlich zu positionieren. Der Patient wird dann mit Hilfe eines Korsetts festgehalten, sodass keine zu Unschärfen der Aufnahme Anlass gebenden Bewegungen möglich sind. Durch die Fixierung erreicht man eine bessere Bildqualität, da die Bewegung des Patienten eingeschränkt ist und weniger Bewegungsartefakte auftreten können.

Schließlich wird vorgeschlagen, die Empfangsspulen über Justiereinrichtungen zu befestigen, die es in der praktischen Anwendung erlauben, die Empfangsspulen optimal auszurichten, um dem zu Folge auch eine bessere Bildqualität zu erzielen. Eine ausdrücklich empfehlenswerte Möglichkeit besteht darin, die Justiereinrichtungen an der Fixiereinrichtung zu befestigen.

In einer vorteilhaften Ausgestaltung wird ein keilförmiges Kissen eingesetzt, das unterhalb des Beckens des Patienten derart eingeschoben wird, dass das Becken leicht nach oben gekippt, was ein Einschieben der Keilspitze in Richtung der Längsachse des Patienten erfordert. Dieses Kissen dient der Ausrichtung des Beckens und damit der Ausrichtung der Prostata zur Empfangsspule. Durch eine optimale Ausrichtung kann eine bessere Bildqualität erreicht werden.

In einer Weiterbildung wird ein Kissen verwandt, das mit Flüssigkeit oder Gas beaufschlagt werden kann, um somit die Form des Kissens zu ändern und damit die Ausrichtung des Beckens des Patienten zu optimieren. Durch eine entsprechende Beaufschlagung des Kissens lassen sich in infinitesimalen Schritten und in weiten Grenzen eine beliebige Ausrichtung des Beckens realisieren.

In einer Ausführungsform kann das Kissen in mehrere Sektoren bzw. Kammern unterteilt sein, welche unterschiedlich mit Gas oder Flüssigkeit beaufschlagt werden können. Wird eine Kammer mit höherem Druck beaufschlagt, vergrößert sie sich, bei geringerem Druck ist die jeweilige Kammer kleiner. Dies hat den Vorteil, dass man zielgenau das Becken des Patienten in unterschiedlichen Raumrichtungen ausrichten kann, indem man die einzelnen Kammern individuell beaufschlagt und damit einstellt. Die Anzahl der Sektoren bzw. Kammern entspricht der Anzahl der Einstellungsparametern, die zur Verfügung stehen.

Ziel ist auch hier die Bildqualität zu verbessern.

Schließlich können auf oder in diesem Kissen auch Empfangsspulen untergebracht sein. Das Verbauen der Empfangsspulen direkt auf oder im Kissen erlaubt eine nähere Platzierung am Patienten, damit verbunden ist eine höhere Auflösung und ein besseres Signal- zu Rausch-Verhältnis.

In einer weiteren Ausgestaltung können für jede Spule elektrische Vorverstärker verbaut sein, um das Signal möglichst schon an der Spule zu verstärken. Dadurch wird die zusätzliche relative Rauschamplitude durch die Leitungen zur Elektronik des magnetischen Resonanz-tomographischen Gerätes kleiner, damit die Signalqualität besser und schließlich auch die Bildqualität.

Im Folgenden soll die Erfindung an Hand von der in der Zeichnung darstellten Ausführungsformen näher beschrieben werden. Es zeigen in prinzipienhaft gehaltenen Darstellungen:
- Figur 1:: ein erfindungsgemäßes Spulenelement mit Patienten in Ansicht
- Figur 2:: Spulenelement mit Patienten sowie eine Fixiereinrichtung

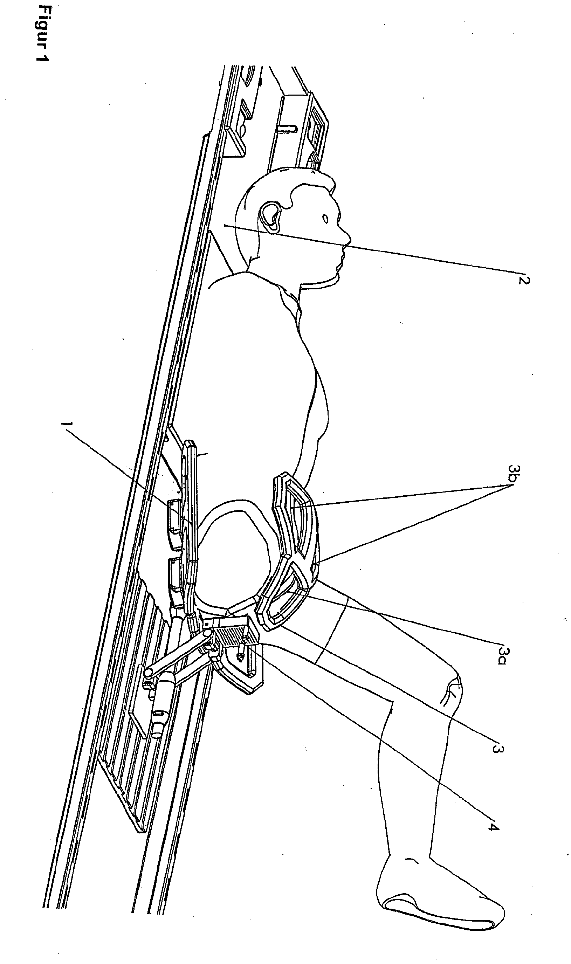

In der 3D-Darstellung der Figur 1 ist schematisch der Patient (2) auf einem Tisch liegend eingezeichnet. Unterhalb des Patienten in der unteren Rückenregion und am Hinterteil befindet sich ein Standardempfangsspulenelement (1), was leicht gebogen ist, so dass es sich etwa dem Rumpf des Patienten anpasst. Das Standardspulenelement (1) besteht typischer Weise aus sechs einzelnen Spulen. Diese Anordnung wird auch Phased-Array genannt.

Auf der unteren Bauchregion und auf dem Leistenbereich des Patienten befindet sich das erfindungsgemäße Spulenelement (3). Dieses ist in drei Teilspulen unterteilt, die dreiecksförmig angeordnet sind. Zwei Spulen (3b) befinden sich parallel nebeneinander auf der unteren Bauchregion des Patienten und sind mit ihrer Flächennormale in Richtung Prostata ausgerichtet. Eine dritte Spule (3a), die in Zusammenhang mit der Erfindung von entscheidender Bedeutung ist, wurde unten an diese zwei Spulen (3b) mittig angefügt, sodass sie Hodensack und Penis umschließt und an den Leisten des Patienten anliegt, wenn dieser die Oberschenkel leicht spreizt. Diese dritte Spule (3a) ist V-förmig gestaltet und ebenfalls mit ihrer Flächennormale in Richtung Prostata ausgerichtet. Die ebenfalls eingezeichnete Biopsieeinrichtung (4) spielt keine Rolle für die Erfindung. Nicht eingezeichnet ist das eigentliche magnetische Resonanztomographische Gerät, d. h. die das starke homogene Magnetfeld erzeugende Spule sowie die Sendespule. Auch nicht dargestellt sind die Gradientenspulen.

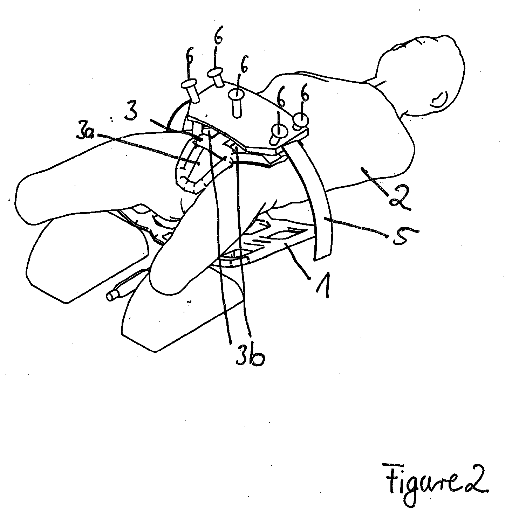

In Figur 2 ist der Patient (2) aus einem im Vergleich zu Figur 1 anderen Blickwinkel schematisch liegend wiedergegeben. Zusätzlich ist eine Justiervorrichtung (5) dargestellt. Unterhalb des Patienten ist ein Standardempfangsspulenelement (1) angebracht, auf welchem der Patient mit der unteren Rückenregion und dem Hinterteil aufliegt. Direkt auf der unteren Bauchdecke und im Leistenbereich des Patienten befindet sich das erfindungsgemäße Spulenelement (3). In Richtung auf den Kopf zu sind die zwei Spulen (3b) auf dem Bauch des Patienten ausgerichtet. In Gegenrichtung schließt die dritte Spule (3a) an, welche Penis und Hodensack umschließt. Die oberhalb des Patienten befindlichen Spulen sind an eine Fixiervorrichtung (5) befestigt, die bogenförmig teilweise den Rumpf des Patienten umschreibt. Oberhalb der zwei oberen Spulen (3b) ist diese Vorrichtung der Form der Spulen angepasst. Fünf Justierschrauben (6) erlauben mittels nicht dargestellter Justiereinrichtungen eine Optimierung der Ausrichtung Spulen (3) relativ zum Patienten (2).

In allen zeichnerischen Darstellungen werden aus Gründen der Klarheit funktionswesentliche Geräteelemente nicht dargestellt. Hierzu zählen die homogene Spule, die Gradientenspulen sowie die zur Auswertung notwendige Datenverarbeitungsanlage.

### Bezugszeichenliste

1 Standardspulenelement
2 Patient
3 Spulen
3a Empfangsspule
3b Spulen
4 Biopsieeinrichtung
5 Fixiereinrichtung
6 Justierschrauben

## Patentansprüche

1. Magnetisches Resonanz-tomographisches Gerät für Prostatauntersuchungen an einem darin befindlichen Patienten (2) bestehend aus einer Spule zur Erzeugung eines starken homogenen magnetischen Feldes in Richtung der Längsachse des Patienten, mindestens einer Sendespule zur Erzeugung eines elektromagnetischen Wechselfeld, drei Gradientenspulen und geeigneten Empfangsspulen (1), von denen einzelne unterhalb in der unteren Rückenregion und/oder am Hinterteil und wenigstens drei oberhalb des Patienten anzuordnen sind, sowie aus einer Datenverarbeitung zur Bildgewinnung aus den Signalen der Sende- und Empfangsspulen, wobei
ein Spulenelement (3) vorgesehen ist, das in drei dreiecksförmig angeordnete Teilspulen (3a, 3b) unterteilt ist und so eingerichtet ist, dass, wenn sich zwei der Teilspulen (3b) parallel nebeneinander auf der unteren Bauchregion des Patienten befinden, eine dritte, geschlossene und mittig angefügte V-förmige Empfangsspule (3a) den Hodensack und den Penis umschließt und an den Leisten des Patienten anliegt, wobei Hodensack und Penis eine Öffnung der Empfangsspule (3a) durchgreifen.

2. Magnetisches Resonanz-Tomographie-Gerät gem. Anspruch 1, **dadurch gekennzeichnet, dass** die die Hoden umgebende Empfangsspule (3a) und/oder die Teilspulen (3b) mit ihrer durch den Flächenschwerpunkt verlaufenden Flächennormalen in Richtung Prostata ausgerichtet ist.

3. Magnetisches Resonanz-Tomographie-Gerät gem. Anspruch 1 oder 2, **dadurch gekennzeichnet, dass** mehr als drei Spulen auf dem Patienten angeordnet sind.

4. Magnetisches Resonanz-Tomographie-Gerät gemäß einem der vorhergehenden Ansprüche, **dadurch gekennzeichnet, dass** die Spulen zu einander beabstandet sind.

5. Magnetisches Resonanz-Tomographie-Gerät gemäß einem der Ansprüche 1 - 3, **dadurch gekennzeichnet, dass** die Spulen direkt aneinander anschließen.

6. Magnetisches Resonanz-Tomographie-Gerät gem. einem der vorhergehenden Ansprüche, **dadurch gekennzeichnet, dass** die Spulen in einer flexiblen Matte untergebracht sind, welche auf dem Patienten liegt.

7. Magnetisches Resonanz-Tomographie-Gerät gem. einem der vorhergehenden Ansprüche, **dadurch gekennzeichnet, dass** der Spulenradius größer oder gleich dem durchschnittlichen Abstand der Spulenebene zur Prostata ist.

8. Magnetisches Resonanz-Tomographie-Gerät gem. einem der vorgehenden Ansprüche, **dadurch gekennzeichnet, dass** eine Fixiereinrichtung (5) bogenförmig den Bauch, den Rumpf des Patienten zumindest teilweise umschließt.

9. Magnetisches Resonanz-Tomographie-Gerät gem. einem der vorhergehenden Ansprüche, **dadurch gekennzeichnet, dass** die Empfangsspulen über Justiereinrichtungen befestigt sind.

10. Magnetisches Resonanz-Tomographie-Gerät gem. einem der vorhergehenden Ansprüche, **dadurch gekennzeichnet, dass** ein keilförmiges Kissen unterhalb des Beckens des Patienten angeordnet ist.

11. Magnetisches Resonanz-Tomographie-Gerät gem. Anspruch 10, **dadurch gekennzeichnet, dass** ein mit Flüssigkeit oder Gas beaufschlagbares Kissen unterhalb des Beckens des Patienten angeordnet ist.

12. Magnetisches Resonanz-Tomographie-Gerät gem. Anspruch 11, **dadurch gekennzeichnet, dass** das Kissen in mehrere Sektoren/Kammern unterteilt ist, welche unterschiedlich mit Flüssigkeit oder Gas beaufschlagbar sind.

13. Magnetisches Resonanz-Tomographie-Gerät gem. einem der Ansprüche 10 bis 12, **dadurch gekennzeichnet, dass** auf oder in dem Kissen Empfangsspulen verbaut sind.

14. Magnetisches Resonanz-Tomographie-Gerät gem. Anspruch 1-6 und 12, **dadurch gekennzeichnet, dass** zu jeder Spule ein elektrischer Vorstärker vorhanden ist.

## Claims

1. Magnetic resonance tomographic device for prostate examinations on a patient (2) located therein, consisting of a coil for generating a strong homogeneous magnetic field in the direction of the longitudinal axis of the patient, at least one transmission coil for generating an electromagnetic alternating field, three gradient coils and suitable receiving coils (1), of which some are to be arranged below in the lower back region and/or on the rear and at least three are to be arranged above the patient, as well as data processing for image acquisition from the signals of the transmission and receiving coils, wherein a coil element (3) is provided, which is divided into three triangularly arranged sub-coils (3a, 3b) and is set up in such a way that when two of the sub-coils (3b) are located parallel to one another on the lower abdominal region of the patient, a third, closed and centrally attached V-shaped receiving coil (3a) encloses the scrotum and the penis and rests against the patient's groins, with the scrotum and penis reaching through an opening in the receiving coil (3a).

2. Magnetic resonance tomographic device according to claim 1, **characterized in that** the receiving coil (3a) enclosing the scrotum and/or the sub-coils (3b) are, with their surface normal going through respective center of area, pointing in the direction of the prostate.

3. Magnetic resonance tomographic device according to claim 1 or 2, **characterized in that** more than three coils are arranged on the patient.

4. Magnetic resonance tomographic device according to one of the preceding claims, **characterized in that** the coils are distanced from one another.

5. Magnetic resonance tomographic device according to one of the claims 1 - 3, **characterized in that** the coils are immediately adjacent to one another.

6. Magnetic resonance tomographic device according to one of the preceding claims, **characterized in that** the coils are housed in a flexible mat which lies on the patient.

7. Magnetic resonance tomographic device according to one of the preceding claims, **characterized in that** the radius of the coils is larger or equal the average distance between the coil plane and the prostate.

8. Magnetic resonance tomographic device according to one of the preceding claims, **characterized in that** a fixing device arcuately encloses stomach, torso of the patient at least partially.

9. Magnetic resonance tomographic device according to one of the preceding claims, **characterized in that** the receiving coil is attached via an adjustment device.

10. Magnetic resonance tomographic device according to one of the preceding claims, **characterized in that** a wedge-shape cushion is arranged below the pelvis of the patient.

11. Magnetic resonance tomographic device according to claim 10, **characterized in that** a cushion which can be filled with liquid or gas, is arranged below the pelvis of the patient.

12. Magnetic resonance tomographic device according to claim 11, **characterized in that** the cushion is subdevided into several sections/chambers, which can be filled with liquid or gas differently.

13. Magnetic resonance tomographic device according to one of the claims 10 to 12, **characterized in that** on or in the cushion receiving coils are installed.

14. Magnetic resonance tomographic device according to claim 1-6 and 12, **characterized in that** a pre-amplifier is present for each coil.

## Revendications

1. Appareil de tomographie par résonance magnétique pour les examens de la prostate sur un patient (2) situé dans celui-ci, constitué d'une bobine pour générer un fort champ magnétique homogène dans la direction de l'axe longitudinal du patient, au moins une bobine de transmission pour générer un champ électromagnétique alternatif, trois bobines à gradient et des bobines de réception appropriées (1), dont certaines doivent être disposées en bas dans la région du bas du dos et/ou à l'arrière et au moins trois doivent être disposées au-dessus de la patient, ainsi que le traitement des données pour l'acquisition d'images à partir des signaux des bobines d'émission et de réception,
dans lequel un élément de bobine (3) est prévu, qui est divisé en trois sous-bobines disposées en triangle (3a, 3b) et est configuré de telle sorte que lorsque deux des sous-bobines (3b) sont situées parallèlement l'une à l'autre sur la région abdominale inférieure du patient, une troisième bobine réceptrice en forme de V fermée et fixée au centre (3a) enferme le scrotum et le pénis et repose contre les aines du patient, le scrotum et le pénis passant par une ouverture dans le bobine réceptrice (3a).

2. Dispositif de tomographie par résonance magnétique selon la revendication 1, **caractérisé en ce que** la bobine de réception (3a) entourant le scrotum et/ou les sous-bobines (3b) sont, avec leur surface normale passant par le centre de la zone respective, pointant dans la direction de la prostate.

3. Appareil de tomographie par résonance magnétique selon la revendication 1 ou 2, **caractérisé en ce que** plus de trois bobines sont disposées sur le patient.

4. Appareil de tomographie par résonance magnétique selon l'une des revendications précédentes, **caractérisé en ce que** les bobines sont distantes l'une de l'autre.

5. Appareil de tomographie par résonance magnétique selon l'une des revendications 1 à 3, **caractérisé en ce que** les bobines sont immédiatement adjacentes l'une à l'autre.

6. Appareil de tomographie par résonance magnétique selon l'une des revendications précédentes, **caractérisé en ce que** les bobines sont logées dans un tapis souple qui repose sur le patient.

7. Appareil de tomographie par résonance magnétique selon l'une des revendications précédentes, **caractérisé en ce que** le rayon des bobines est supérieur ou égal à la distance moyenne entre le plan des bobines et la prostate.

8. Appareil de tomographie par résonance magnétique selon l'une des revendications précédentes, **caractérisé en ce qu'** un dispositif de fixation entoure en arc de cercle l'estomac, le torse du patient au moins partiellement.

9. Appareil de tomographie par résonance magnétique selon l'une des revendications précédentes, **caractérisé en ce que** la bobine de réception est fixée via un dispositif de réglage.

10. Appareil de tomographie par résonance magnétique selon l'une des revendications précédentes, **caractérisé en ce qu'** un coussin en forme de coin est disposé sous le bassin du patient.

11. Appareil de tomographie par résonance magnétique selon la revendication 10, **caractérisé en ce qu' un** coussin pouvant être rempli de liquide ou de gaz est disposé sous le bassin du patient.

12. Appareil de tomographie par résonance magnétique selon la revendication 11, **caractérisé en ce que** le coussin est subdivisé en plusieurs sections/chambres, qui peuvent être remplies différemment de liquide ou de gaz.

13. Appareil de tomographie par résonance magnétique selon l'une des revendications 10 à 12, **caractérisé en ce que** sur ou dans le coussin des bobines de réception sont installées.

14. Dispositif de tomographie par résonance magnétique selon les revendications 1 à 6 et 12, **caractérisé en ce qu**'un préamplificateur est présent pour chaque bobine.
